(19)

Europäisches
Patentamt
European
Patent Office
Office européen
des brevets

(11) **EP 1 774 902 A1**

(12) **EUROPEAN PATENT APPLICATION**

(43) Date of publication:
**18.04.2007 Bulletin 2007/16**

(51) Int Cl.:
***A61B 5/00*** (2006.01)

(21) Application number: **05028512.1**

(22) Date of filing: **27.12.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **14.10.2005 JP 2005300962**

(71) Applicant: **HITACHI, LTD.**
**Chiyoda-ku**
**Tokyo 100-8280 (JP)**

(72) Inventors:
• **Nagata, Koji, c/o Hitachi, Ltd.**
**Tokyo 100-8220 (JP)**

• **Mitsumaki, Hiroshi, c/o Hitachi, Ltd.**
**Tokyo 100-8220 (JP)**
• **Uchida, Tsuyoshi, c/o Hitachi, Ltd.**
**Tokyo 100-8220 (JP)**
• **Asano, Rinichi**
**Hitachi-shi**
**Ibaraki 317-0073 (JP)**
• **Kiguchi, Masashi, c/o Hitachi, Ltd.**
**Tokyo 100-8220 (JP)**

(74) Representative: **Strehl Schübel-Hopf & Partner**
**Maximilianstrasse 54**
**80538 München (DE)**

(54) **Blood glucose measurement device and metabolic rate measurement device**

(57) Measurement of blood flow rate in a convenient manner has been enabled by using a simple device constitution and a simple signal processing technique. A measurement mechanism having both the function of measuring temperature of the measurement site and the function of applying a thermal load to the measurement site is used, and a process of extracting temperature change associated with the recovery of the surface temperature by the blood flow is conducted from the measurement results obtained by the measurement mechanism.

FIG. 5 B

EP 1 774 902 A1

**Description**

CLAIM OF PRIORITY

The present application claims priority from Japanese application JP 2005-300962 filed on October 14, 2005, the content of which is hereby incorporated by reference into this application.

FIELD OF THE INVENTION

This invention relates to a device capable of measuring blood glucose level or metabolic rate of a skin system in a non-invasive and simple manner by using a percutaneous blood flow rate measurement technique.

BACKGROUND OF THE INVENTION

Two known methods, namely, a method using laser irradiation and a method using heat conduction method may be used in percuataneously measuring the blood flow rate of a skin tissue. An example of the former method is JP No.3478346 in which the tissue blood flow rate is measured by using a laser beam. In this method, the skin at the site of the measurement is irradiated with a laser beam, and frequency of the multiple intensity change found in the signal intensity of the reflected and scattered light beam is analyzed to calculate the blood flow rate. As an example of the latter method, JP-A No. 248267/1995 discloses a method in which a heat conducting block and a thermocouple are used to measure the temperature of an internal surface. In the use of this method, a heating point and a nearby temperature measurement point are provided on the skin surface of the measurement site, and the heating point is heated simultaneously with the measurement of the temperature at the temperature measurement point so that these points are under feed back control of the heat, and the skin surface temperature is kept at a constant level. In this measurement, the blood flow rate is calculated based on the principle that the change in the heat applied to the heating point is proportional to the blood flow rate underneath the skin at that point.

SUMMARY OF THE INVENTION

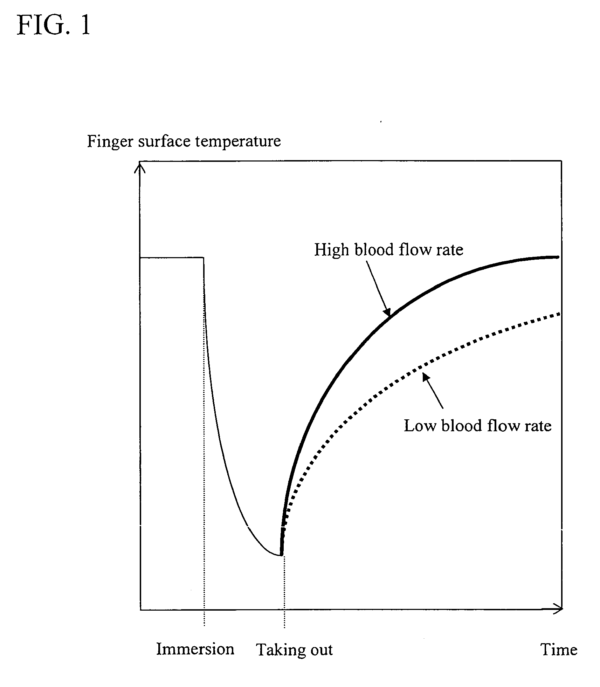

The methods as described above are the methods commonly used in measuring the blood flow rate. Both of these methods, however, require a complicated setup of the measurement device or an advanced signal processing technology for the processing of the measured signals, and this led to the large device size and a high operational cost.
In view of the problems associated with the prior art device as described above, an object of the present invention is to provide a device capable of measuring the blood glucose level or the metabolic rate by using a simple device constitution and simple signal processing technology, in which the blood flow rate can be non-invasively measured in a convenient manner and the blood glucose level or the metabolic rate can be determined from the thus obtained blood flow rate information in a convenient manner.
Ying, He (Riken) et al. "Numerical and Experimental Study on the Human Blood Circulation and Heat Transport Phenomena" has reported that recovery process of the surface temperature after applying a thermal load to a finger, for example, by immersing the finger in a cold water depends on the blood flow rate ($\omega$) in the interior of the finger. When an experiment of applying a thermal load to a finger is conducted by immersing the finger in its thermal equilibrium state in a cold water for a predetermined time, and taking out the finger from the cold water and resting the finger at room temperature, a finger surface temperature profile as shown FIG. 1 is obtained. When the finger is immersed in the cold water, the temperature that had once been in its thermal equilibrium decreases. When the finger is taken out of the cold water, the finger gradually warms and finally recovers its initial temperature. Blood flow rate affects such recovery process, and a difference in the temperature recovery rate as shown in FIG. 1 generates in the recovery process depending on the blood flow rate. This phenomenon suggests that the rate of the blood flow in the interior of the finger can be estimated by using the change of the finger surface temperature caused upon application of the thermal load (cooling), and in particular, by using the profile of the finger surface temperature measured after completing the cooling. In the blood flow rate measurement method of the present invention, use of such phenomenon in the measurement has been enabled by providing a measurement mechanism having both the mechanism of measuring the temperature and the mechanism of applying the thermal load, and processing the measured data obtained in the measurement mechanism to thereby extract the part corresponding to the temperature change caused by the measurements obtained by the above-described phenomenon. The thus obtained blood flow rate information is used in the measurement of the blood glucose level or the metabolic rate.
According to one aspect of the present invention, a blood glucose measurement device for measuring blood glucose level comprises a heat measurement unit for measuring a plurality of temperatures from body surface to obtain information used in calculating amount of the heat transmitted by convection and radiation in heat dissipation from the body surface; an oxygen content measuring unit for obtaining information on blood oxygen content; a storage unit for storing the relations between parameters respectively corresponding to the plurality of temperatures and the blood oxygen content and the blood glucose level; an arithmetic unit for converting the plurality of measurements received from the heat measurement unit and the oxygen content measuring unit respectively into the parameters, and calculating the blood glucose levels by applying the parameters to the relations stored in the storage unit; and a display unit for displaying the results obtained in the arithmetic unit; wherein the oxygen content measuring unit comprises a unit for measuring blood flow rate to obtain information on the blood flow rate; and an optical measurement unit for obtaining blood hemoglobin concentration and hemoglobin oxygen saturation; and the blood flow rate measuring unit comprises a member to be brought in contact with body surface; a temperature sensor provided in contact with the body surface contacting member at a surface opposite to the side to be brought in contact with the body surface; a metal block; and a thermal connection member for thermally connecting the temperature sensor and the metal block.

According to another aspect of the present invention, a blood glucose measurement device for measuring blood glucose level comprises an ambient temperature measurement device for measuring ambient temperature; a body surface contacting unit to be brought in contact with a body surface; a radiant heat sensor for measuring heat radiated from the body surface; a temperature sensor provided in contact with the body surface contacting member on a surface opposite to the side to be brought in contact with the body surface; a metal block; a thermal connection member for thermally connecting the temperature sensor and the metal block; a light source for emitting at least two beams respectively having different wavelengths to the body surface contacting member; a photodetector for sensing the beam that had been directed to the body surface; an arithmetic unit having a conversion section for converting outputs from the ambient temperature measurement device, the radiant heat detector, the temperature sensor, and the photodetector respectively to the corresponding parameters, and an arithmetic section which has preliminarily stored relations between the parameters and the blood glucose level and which calculates the blood glucose level by applying the parameters to the relationships; and a display unit for displaying the output of the arithmetic section.

According to another aspect of the present invention, a metabolic rate measurement device comprises an ambient temperature measurement device for measuring the ambient temperature; a first temperature measurement device for measuring temperature of a first body surface site of the subject; a second temperature acquiring unit for acquiring temperature of a second body surface site of the subject which is different from the first body surface site; a blood flow rate acquiring unit for acquiring information on blood flow rate of the first site; a storage unit for storing the relations between the ambient temperature, the temperature of the first site, the temperature of the second site, and the blood flow rate and the metabolic rate; an arithmetic unit for calculating the metabolic rate by applying the ambient temperature measured by the ambient temperature measurement device, the temperature of the first site measured by the first temperature measurement device, the temperature of the second site measured by the second temperature measurement device, and the blood flow rate acquired by the blood flow rate acquiring unit to the relations stored in the storage unit; and a display unit for displaying the results calculated in the arithmetic section; wherein the blood flow rate acquiring unit comprises a body surface contacting section which contacts with the first body surface site, a temperature sensor provided in contact with the body surface contacting member on a surface opposite to the side to be brought in contact with the body surface, a metal block, and a thermal connection member for thermally connecting the temperature sensor and the metal block.

According to another aspect of the present invention, a metabolic rate measurement device comprises an ambient temperature measurement device for measuring the ambient temperature; a body surface contacting unit which contacts with a first body surface site; a cylindrical member which is in contact with the body surface contacting unit and which is open at one end; a radiant heat thermometer for measuring the radiant heat emitted from the first site, the radiant heat thermometer being provided near the end of the cylindrical member other than the open end; a blood flow rate acquiring unit for acquiring information on the blood flow rate of the first site, the unit comprising a temperature sensor provided in contact with the body surface contacting unit at a surface opposite to the side to be brought in contact with the body surface, a metal block, and a thermal connection member for thermally connecting the temperature sensor and the metal block, and the information on the blood flow rate of the first site is acquired from temperature change of the body surface contacting unit after being brought in contact with the body surface first site; a light source which emits at least two light beams each having different wavelengths to the end of the cylindrical member; a photodetector for detecting the light beam which has interacted with the body surface; a second site temperature acquiring unit for acquiring temperature of the second site which is different from the first site an arithmetic unit wherein the ambient temperature measured by the ambient temperature measurement device, the temperature of the first site measured by the first temperature measurement device, the temperature of the second site acquired by the second temperature acquiring unit, the blood flow rate acquired by the blood flow rate acquiring unit, and the results measured by the photodetector are applied to preliminarily stored relations between these values and the metabolic rate to thereby calculate the metabolic rate; and a display unit for displaying the output of the arithmetic section. The thermal connection member has a thermal conductivity lower than that of the metal block, and the metal block has a heat capacity of at least $3.0 \times 10^{-3}$ (KJ/K).

The present invention has enabled to measure blood glucose level and metabolic rate in a convenient manner by a simple device constitution and by a simple signal processing technique.

BRIEF DESCRIPTION OF THE DRAWINGS

FIG. 1 is a view presented for explaining the relation between the change in the finger surface temperature and the blood flow.
FIG. 2 is a view presented for explaining heat conduction model of a living body.
FIG. 3 is another view presented for explaining the heat conduction model of the living body.
FIG. 4 is a view presented for explaining the heat conduction model of the living body and the heat conduction model of the measurement device.
FIG. 5 is a view presented for explaining an embodiment of the present invention. FIG. 5A is a top view, and FIG. 5B is a cross-sectional view.
FIG. 6 is a view presented for explaining a signal waveform measured in the present invention.
FIG. 7 is a view presented for explaining another embodiment of the present invention.
FIG. 8 is another view presented for explaining another embodiment of the present invention.

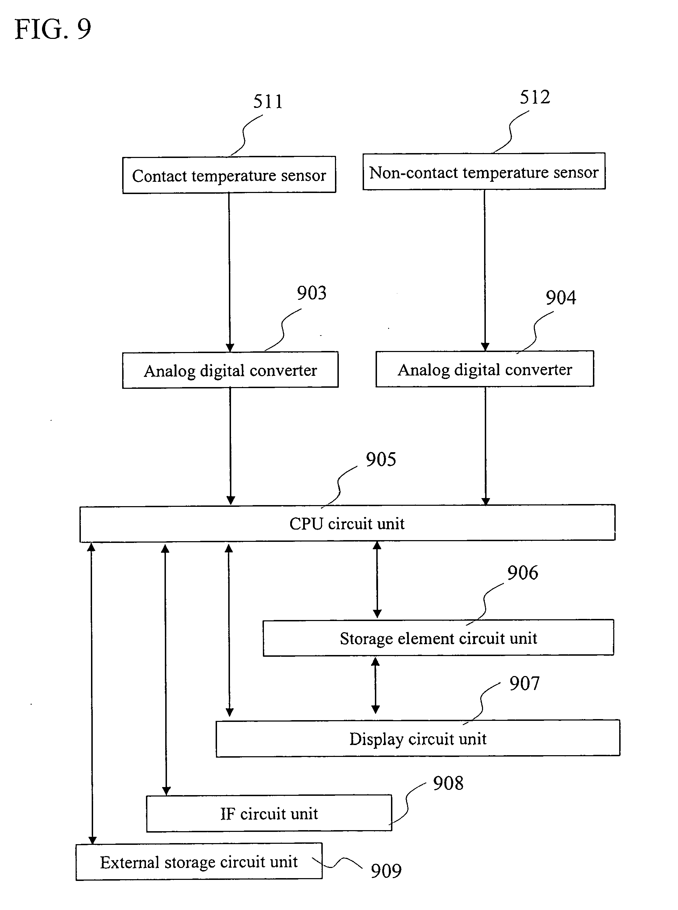

FIG. 9 is another view presented for explaining another embodiment of the present invention.
FIG. 10 is a view presented for explaining another embodiment in which the present invention has been applied for blood glucose level estimation.
FIG. 11 is a view presented for explaining an embodiment of the measurement conducted by the present invention.
FIG. 12 is a view presented for explaining the measurement conducted to measure blood glucose level.
FIG. 13 is a view presented for explaining another embodiment in which the present invention has been applied for blood glucose level measurement. FIG. 13A is a top view, and FIGS. 13B and 13C are side views.
FIG. 14 is a top view of a metabolism meter according to the present invention.
FIG. 15 is a top view of another metabolism meter according to the present invention.
FIG. 16 is a view presented for explaining the input operation of numerical data.
FIG. 17 is a view presented for explaining an embodiment in which blood flow rate measurement method of the present invention has been applied for metabolic rate measurement.

DETAILED DESCRIPTION OF THE PREFERRED EMBODIMENTS

Pennes equation as cited below is a widely known equation describing the heat conduction in a living body such as finger including the blood flow (Pennes, H. H., "Analysis of tissue and arterial blood temperatures in the resting human forearm", J. Applied Physiology, Vol.1, No.2, pp.93-122, 1948).

$$\rho_t c_t \frac{\partial T_t}{\partial t} = \lambda_0 \nabla^2 T_t + q_m + \omega_b \rho_b c_b (T_a - T_t)$$

In Pennes equation, temperature change of the tissue at an arbitrary point is expressed by a component proportional to the temperature difference (Ta-Tt) between blood temperature Ta and the tissue temperature Tt and blood flow rate $\omega$, a component reflecting the temperature difference between adjacent tissues (spatial temperature distribution), and furthermore, local thermogenesis (metabolic heat).

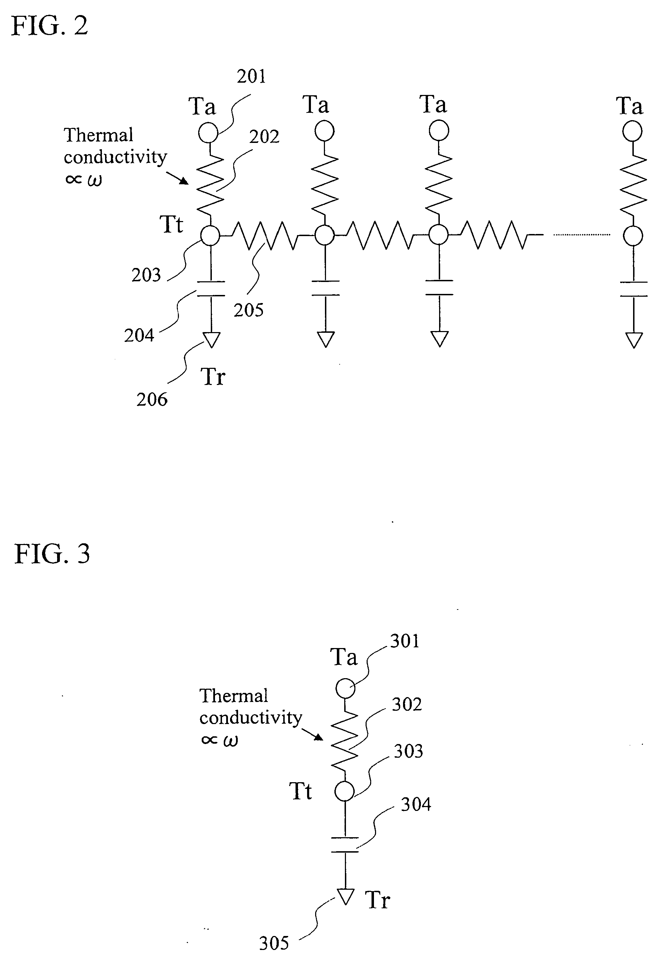

FIG. 2 is this relation expressed in terms of a heat conduction circuitry. In this circuitry, the term of the metabolic heat has been omitted to limit the explanation to a site such as finger. The expression of two dimensional and three dimensional circuitry are also omitted. All temperatures are expressed in terms of difference from room temperature Tr by using the room temperature Tr for the referential temperature. In this heat conduction circuitry of FIG. 2, the heat source is blood temperature Ta, and the heat conduction pathway is defined as a pathway determined by each tissue site and the proportional relation as described above. When this thermal pathway is compared with Fourier law which is the general equation describing the heat conduction, blood flow rate $\omega$ can be treated as the thermal conductivity between the site having the blood temperature Ta and each tissue site, and the thermal conductivity and the thermal resistance in the heat conduction circuitry are in reverse proportional relation. As a consequence, the heat conduction circuitry of FIG. 2 can be expressed by postulating a site 201 having the blood temperature Ta, and by expressing the site 201 as being connected to each tissue site 203 by a thermal resistance 202 which depends on the blood flow $\omega$. Thermal resistance 205 which depends on the thermal conductivity between the tissue sites is defined between tissue sites 203, and heat capacity 204 of each tissue site is also defined.

In the measurement of the blood flow rate according to the present invention, the size of the region to which the thermal load is applied to the entire tissue such as finger and the size of the region carrying out the temperature measurement are minimized to the level that the entire tissue such as finger can be deemed as a semi-infinitely homogeneous substance and the heat conducting circuitry can be expressed in terms of a lumped constant. Therefore, the heat conduction circuitry of FIG. 2 can be simplified as the heat conduction circuitry of FIG. 3. In FIG. 3, 301 represents the blood temperature, 302 represents the thermal resistance, 303 represents the tissue site, 304 represents the tissue heat capacity, and 305 represents the room temperature. In this case, because of the reason for the simplification as described above, no temperature difference, and hence, no heat transfer is found between the lumped tissue sites, and therefore, the thermal resistance representing the heat conduction pathway is negligible. In the following section, the blood flow rate measuring method of the present invention is described by assuming that the heat conduction circuitry in the tissue of the measurement site in the form as shown in FIG. 3.

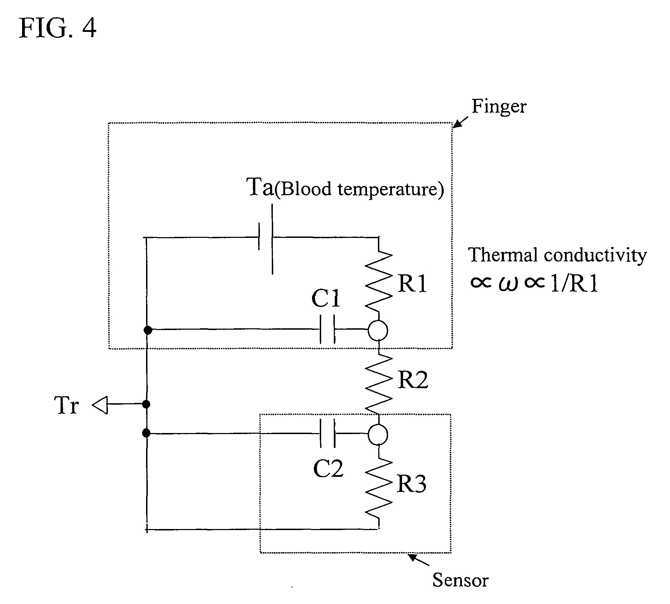

FIG. 4 is a view presented for explaining the device when the site to be measured is a fingertip. In FIG. 4, $T_a$ represents the blood temperature, $T_r$ represents the room temperature, $R_1$ represents the thermal resistance which is reverse proportional to the blood flow $\omega$, contact thermal resistance $R_2$ between the sensor and the finger, $R_3$ is thermal resistance between the sensor and the room temperature, $C_1$ is the heat capacity of the tissue, and $C_2$ is the heat capacity of the sensor. The device of FIG. 4 adopts a system in which a contact temperature sensor (for example, a thermistor) is brought in contact with the measurement site. In the blood flow measurement of the present invention, the necessary condition is that the finger is in equilibrium with the room temperature $T_r$, and this is the initial state. In this initial state, heat from the core temperature $T_a$ is transferred by blood flow $\omega$ to be accumulated by the heat capacity $C_1$ of the finger tissue, and the surface temperature $T_s$ has been stabilized to an initial value $T_{s0}$.

This initial value of the finger surface temperature is determined by the blood temperature $T_a$, the thermal resistance $R_1$ which is reverse proportional to the blood flow $\omega$, and the thermal resistance $R_{air}$ between the finger surface and the air determined by the heat conduction coefficient from the finger surface to the air, as shown below.

$$T_{S0} = \frac{R_{air}}{R_{air} + R_1} T_a$$

When the sensor is brought in contact with the finger in such constant state, the heat accumulated in the tissue heat capacity $C_1$ transfers to the sensor. The change in the finger surface temperature $T_s$ and the sensor (thermistor) temperature $T_1$ associated with this heat transfer are represented by the following equation.

$$T_S(t) = T_{S0}(1 - \frac{c_2}{c_1 + c_2}(1 - e^{-\frac{c_1+c_2}{R_2 c_1 c_2}t}))$$

$$T_1(t) = T_{S0} \frac{c_1}{c_1 + c_2}(1 - e^{-\frac{c_1+c_2}{R_2 c_1 c_2}t})$$

The sensor then becomes thermally saturated in a short time by the heat transferred from the finger, while the finger surface temperature is reduced, and this means that a thermal load is given to the finger surface. The sensor of the present invention has been designed to reduce the contact thermal resistance $R_2$ between the sensor and the finger by using a contact temperature sensor surface having a high thermal conductivity. In addition, a heat capacity is provided on the contact temperature sensor on the surface opposite to the surface that is brought in contact with the measurement site to thereby facilitate transfer of the heat in a short period and stabilize the temperature at such position.

Simultaneously with the process of the thermal saturation of the sensor, the finger proceeds to the process of heat re-accumulation (temperature recovery process) in the heat capacity $C_1$ from the heat source in the deeper portion of the finger transferred through the blood flow. The temperature change in this process is represented by the following equation. Since the sensor has already been thermally saturated as described above, this temperature recovery process can be measured without being influenced by the response property of the sensor.

$$T_1(t) = R_3\left[\frac{T_a}{R_1 + R_2 + R_3} + \left(\frac{T_S}{R_2 + R_3} - \frac{T_a}{R_1 + R_2 + R_3}\right)\exp\left(-\frac{R_1 + R_2 + R_3}{C_1 R_1 (R_2 + R_3)}t\right)\right]$$

Accordingly, the waveform of the temperature detected by the sensor of the present invention when it is brought in contact with the finger mainly comprises the change represented by the following equation in the period between immediately after the contact of the sensor with the finger and the thermal saturation of the sensor.

$$T_1(t) = T_{S0}\frac{c_1}{c_1 + c_2}(1 - e^{-\frac{c_1 + c_2}{R_2 c_1 c_2}t})$$

After the thermal saturation, the waveform mainly comprises the change represented by the following equation.

$$T_1(t) = R_3\left[\frac{T_a}{R_1 + R_2 + R_3} + \left(\frac{T_S}{R_2 + R_3} - \frac{T_a}{R_1 + R_2 + R_3}\right)\exp\left(-\frac{R_1 + R_2 + R_3}{C_1 R_1 (R_2 + R_3)}t\right)\right]$$

In the blood flow measurement of the present invention, focus is on the temperature recovery process, namely, the change after the thermal saturation.

Differentiation of the temperature change obtained from the temperature recovery process, namely, during the temperature change after the thermal saturation is differentiated with respect to time gives the following equation. This equation indicates that the time differentiation in the temperature recovery process, namely, the thermal saturation after the thermal saturation is a product of the component proportional to the blood flow rate and the initial finger surface temperature ($T_{s0}$).

$$\frac{dT_1}{dt} \doteqdot \frac{R_3 T_a}{c_1 R_1 (R_2 + R_3)} - \frac{R_3 T_{S0}}{R_1 (R_2 + R_3)(c_1 + c_2)} - \frac{R_3 T_{S0}}{(R_2 + R_3)^2 (c_1 + c_2)}$$

$$\doteqdot \left\{\left[\frac{k}{c_1 (R_2 + R_3)} - \frac{1}{(R_2 + R_3)(c_1 + c_2)}\right]BF - \frac{1}{(R_2 + R_3)^2 (c_1 + c_2)}\right\}R_3 T_{S0}$$

Division of this by initial finger surface temperature ($T_{s0}$) gives a value which is proportional to the blood flow BF($\omega$) having a factor of proportionality which is determined by (1) a fixed value representing the finger properties and (2) a fixed value representing the properties of the sensor.

$$\left\{\left[\frac{1}{C_1 (R_2 + R_3)} - \frac{1}{(R_2 + R_3)(C_1 + C_2)}\right]BF + \frac{1}{C_1 (R_2 + R_3)R_{air}} - \frac{1}{(R_2 + R_3)^2 (C_1 + C_2)}\right\}R_3$$

As described above, the blood flow measuring method of the present invention is capable of measuring the blood flow rate of the measurement site by applying a thermal load to the measurement site on the finger surface by bringing the temperature sensor in contact with the measurement site, and subsequently measuring the temperature recovery process of measurement the site.

Example 1

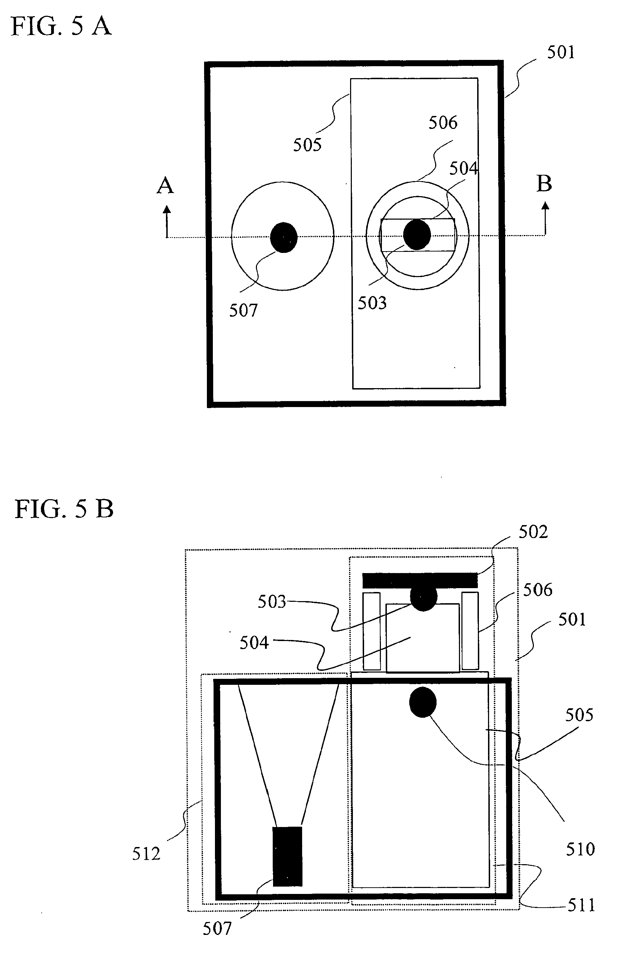

As described above, a value which is proportional to blood flow rate can be measured when initial temperature of a part such as a fingertip of the human at rest and in thermal equilibrium and the room temperature are first measured and recovery of the skin surface temperature is measured after applying a thermal load to such site. In other words, the minimum data that should be collected are the initial finger temperature, the room temperature, and the change of skin surface temperature. An embodiment of the blood flow measurement system of the present invention based on such principle is shown in FIG. 5. FIG. 5A is a top view of the device, and FIG. 5B is a cross-sectional view taken along lines AB. In this Example, a sensor 501 comprises a contact temperature sensor 511 aligned with a non-contact temperature sensor 512. The contact temperature sensor 511 is the site where the recovery stage is measured, and also, the site by which the thermal load is applied. The non-contact temperature sensor 512 is provided mainly for measuring the initial value of the finger surface temperature when nothing has touched the finger surface, and it comprises an infrared sensor 507.

The contact temperature sensor 511 has the structure comprising a contact member 502 formed from a thin layer of a metal having a high thermal conductivity such as gold; a temperature sensor 503 provided on the contact member 502 on the side opposite to the side to be brought in contact with the measurement site; a metal block 505; a support member 504 for thermally connecting the temperature sensor 503 and the metal block 505; and a heat insulating cover member 506. The support member 504 is the route of heat transfer, and it preferably has a thermal conductivity lower than that of the metal block 505 in order to thermally separate the metal block 505 and the temperature sensor 503. The support member 504 typically comprises a material having a thermal conductivity of up to 5 W/m/K.

The metal block 505 is provided in order to maintain and stabilize the standard temperature of the contact temperature sensor 511 at room temperature throughout the measurement period. In view of such an object, a lower limit is set for the metal block 505. In order to maintain the temperature fluctuation of the reference point within 0.5°C throughout the measurement period, the metal block 505 should have a heat capacity of at least $3.0 \times 10^{-3}$ (kJ/K). And this corresponds to a mass of about 10 g when the metal employed is copper. While the mass required may vary depending on the physical properties of the metal, the metals are equivalent as long as the heat capacity is within such a range. In this example, a temperature sensor 510 is provided to monitor the temperature of the metal block 505. While the data obtained by the temperature sensor 510 are not directly used in the measurement of the blood flow, they provide a significant clue in estimating the thermal situation of the sensor.

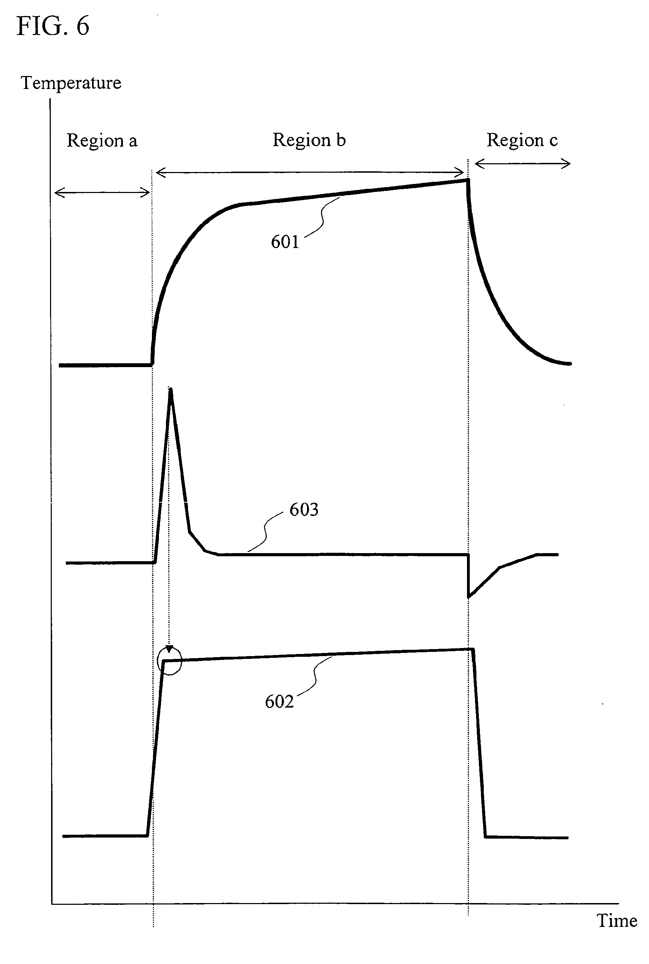

FIG. 6 shows a typical temperature waveform detected by the contact temperature sensor of this embodiment when the site measured is a fingertip. The horizontal axis shows the time, and the vertical axis shows the temperature or the temperature differentiated in relation to time.

Region "a" corresponds the state before the contact of the contact temperature sensor 511 with the finger, and the temperature being detected is the room temperature. Region "b" corresponds to the time zone when the contact temperature sensor 511 is in contact with the finger. Signal waveform 601 of the contact temperature sensor saturates at a response time constant determined by heat capacity of the tissue, heat capacity of the sensor, and the like, and after that, the waveform will reflects the change of the finger surface temperature. On the other hand, the non-contact temperature sensor 512 will immediately reflect the surface temperature as soon as the measurement site is brought into the sensor field as shown in signal waveform 602, and with a higher responsiveness. Region c is the time zone after separating the finger from the sensor. The output of the contact temperature sensor 511 slowly resumes its initial state (room temperature) by dissipating the heat that had accumulated in this sensor. The output of the non-contact temperature sensor 512 immediately resumes its initial state as soon as the measurement site is out of the sensor field.

In the present invention, the thus obtained waveforms 601 and 602 are processed by the principle as described above to thereby calculate the blood flow rate. First, the room temperature is calculated from the results of the region "a". An exemplary calculation method is to find the average value for the entire region "a" of the waveform 601.

Next, initial value of the finger surface temperature is calculated from the signal waveform 602 of the non-contact temperature sensor in region "b". An exemplary calculation method is to use the output of the non-contact temperature sensor at the time when differentiated value 603 of the signal waveform 601 of the contact temperature sensor is at its maximum. This is because the time when the differentiated value of the waveform detected for the contact temperature sensor is at its maximum indicates the actual contact time of the finger with the sensor, and also, the time when the distance between the contact temperature sensor and the object being measured is most optimal.

Next, the non-linear least square method of the following equation is applied to the region "b" of the waveform 601 of the contact temperature sensor to calculate coefficient B1 of the linear function portion of the equation.

$$Temperature = B_0 + B_1 t + B_2 \exp(B_3 t) \qquad \cdots (1)$$

As described above, the temperature waveform detected in the present invention includes stage from the contact of the sensor to the thermal saturation when the temperature increases as an exponential function (short time constant), and the recovery stage when the temperature increases as an exponential function (long time constant). In equation (1), the increase from the contact of the sensor to the thermal saturation as an exponential function (short time constant) is represented as an exponential function having coefficients $B_2$ and $B_3$, and the increase as an exponential function in the subsequent recovery stage (long time constant) is approximated as a linear function having the coefficient $B_1$.

In the equation, the temperature is the value of the signal waveform 601, and t is the time. The functional forms used in the least square method are not limited to those described above as long as the sensor saturation stage and the temperature recovery stage are separately treated.

In the relation as described above, the coefficient $B_1$ is the differential coefficient when the increase as exponential function in the recovery stage (long time constant) is approximated as a linear function, and this coefficient $B_1$ is equal to the product of the component proportional to the blood flow rate and the difference between the initial finger temperature and the room temperature.

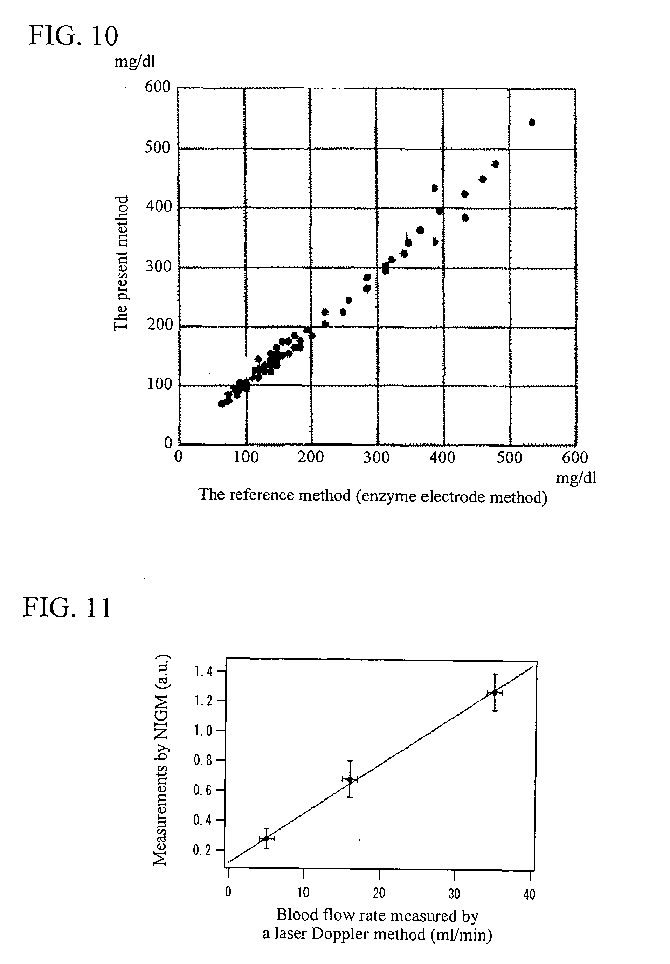

Therefore, a value proportional to the blood flow rate can be obtained by dividing the coefficient $B_1$ of the linear function portion that has been calculated as described above by the difference between the initial finger temperature and the room temperature that has been calculated as described above. FIG. 11 shows the measurements of the blood flow rate measurement device of the present invention plotted in relation to the measurements of laser blood flow rate measurement device used as the reference (horizontal axis). As demonstrated above, blood flow rate ($\omega b$) can be measured by calibrating the value obtained with a conventional blood flow rate measurement device such as a laser device.

It is also evident that the procedure as described may be conducted by using the region "b" of the waveform 602 of the non-contact temperature sensor for the waveform to which the least square method is applied. The calculation of the room temperature and the calculation of the finger surface temperature is also not limited to the embodiments as described above. In addition, the temperature sensor used for measuring the room temperature and the like may also be provided separately from the contact temperature sensor and the non-contact temperature sensor.

FIG. 5 shows an embodiment of the measurement circuit block. The signal from each temperature sensor (the contact temperature sensor 511 or the non-contact temperature sensor 512) is converted by an analog digital converter 903 or 904 into a digital signal. The conversion period is sufficiently shorter than the time resolution of the measurement, and the conversion period in this embodiment is 0.1 sec. The converted data is then processed as described above in a CPU circuit unit 905 and a storage element circuit unit 906. The results are shown numerically in display circuit unit 907. A IF circuit unit 908 is where the operator (user) conducts various operations such as starting and finishing of the processing. The results are stored in an external storage circuit unit 909.

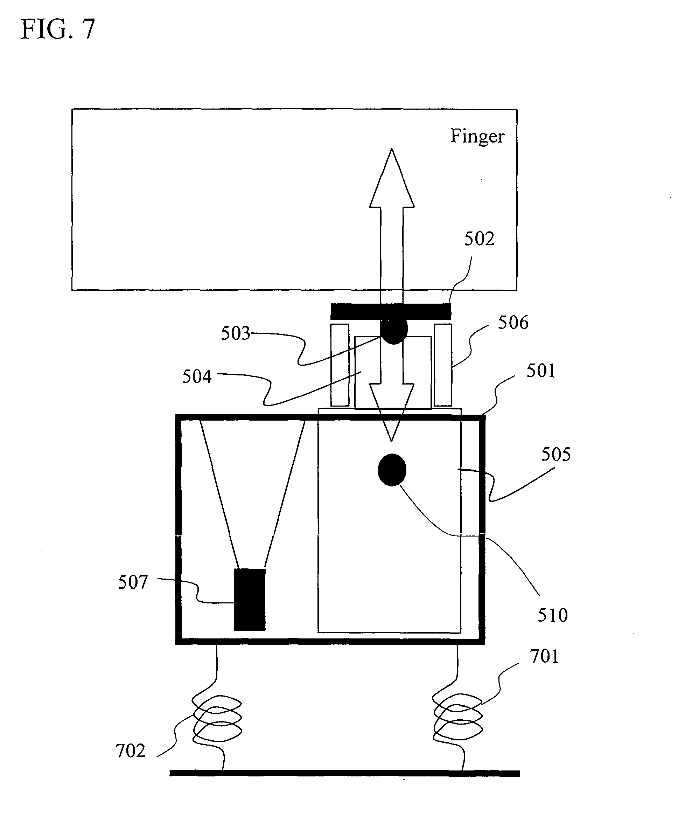

FIG. 7 shows a modification of the measurement device of the present invention. In the measurement device of FIG. 7, the measurement device has been modified by allowing movement of the temperature sensor of FIG. 5 in the direction of the action of the load applied upon contact with the site to be measured such as finger. As shown in FIG. 7, a load is created in the measurement by the action and the reaction between the sensor and the measurement site (in this case, the finger). The force applied to the finger presses the tissue and suppresses the blood flow at the site, and this may adversely affect the precision of the measurement. In this Example, the device has a structure such that the entire sensor can move in the direction of the load application so that the finger contact pressure is controlled by the action of the springs 701 and 702, and the like. Accordingly, in this Example, a reduced pressure will be applied to the site of measurement with reduced degree of blood flow suppression, and the measurement at a high precision is thereby enabled.

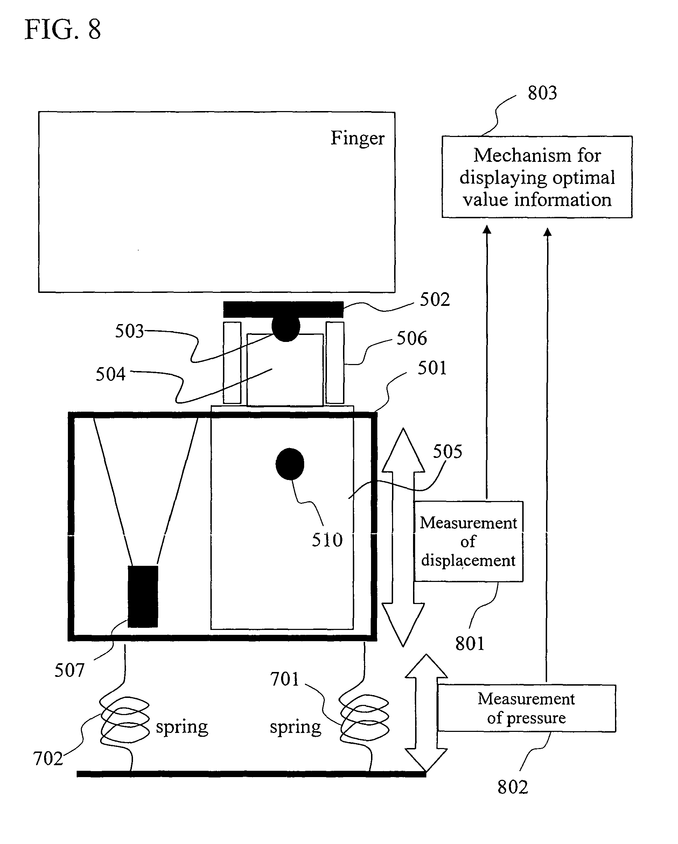

FIG. 8 shows another modification of the measurement device of the present invention. In the measurement device of FIG. 8, the measurement device has been modified by enabling displacement of the entire temperature sensor of FIG. 5 in the direction of the action of the load, and the contact pressure is controlled by the action of springs 701 and 702, and the like. The user is also informed of the appropriate pressure since the device is provided with a mechanism of measuring the displacement or detecting the position of the movable portion 801 or a mechanism of measuring the load 802 together with a mechanism of displaying the optimal value information 803. The mechanism of displaying the optimal value information 803 determines whether the sensor is pressed by the site of the measurement such as the finger at an adequate pressure or by an excessive or insufficient force based on the output of the mechanism 801 or the mechanism 802, and displays the thus determined result. In this Example, a reduced pressure will be applied to the site of measurement, and also, the operator will be allowed to conduct the measurement with the optimal pressure informed, and accordingly, the blood flow will be suppressed to a reduced degree to enable the measurement at a high precision.

Example 2

An example of applying the measurement system of the present invention to the measurement of blood glucose level is described. Japanese Patent Laid-Open No. 2004-329542 discloses that blood glucose level (Glu) can be estimated from a polynomial expression (2) containing n parameters including the parameter representing the blood flow rate.

$$Glu = f(x1, x2, x3, x4, ...., xn) \qquad \cdots(2)$$

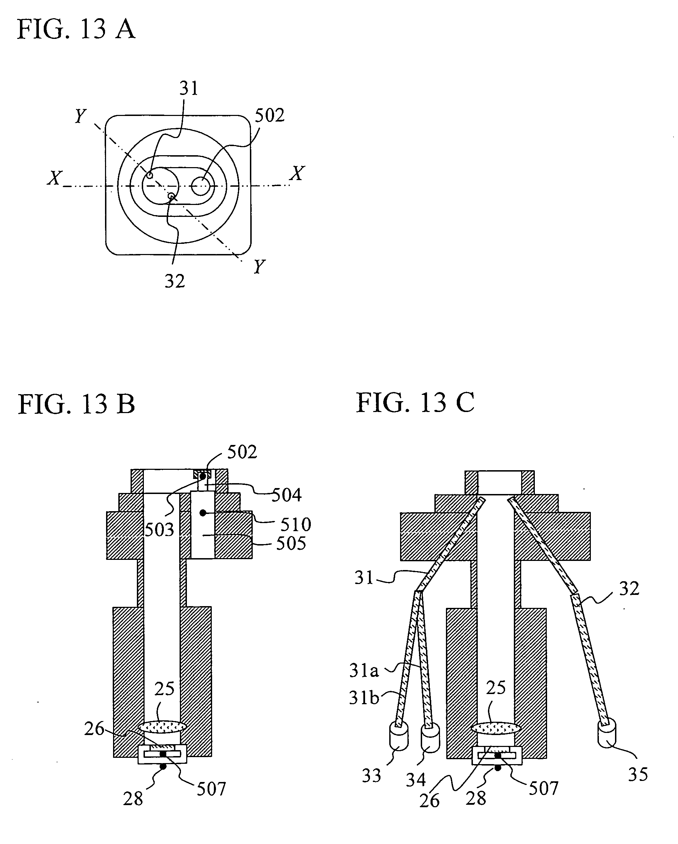

FIG. 13 is a view showing the details when the unit for measuring blood flow rate shown in FIG. 5 of the present invention is applied to the non-invasive blood glucose measurement device disclosed in Japanese Patent Application Laid-Open No. 2004-329542, supra. FIG. 13A is a top view, FIG. 13B is a cross-sectional view taken along line X-X, and FIG. 13C is a cross-sectional view taken along line Y-Y.

The temperature measurement unit of a non-invasive blood glucose measurement device described in this Example is the unit measuring the blood flow rate as described above, and the structure and the measurement principle are as described in Example 1.

Next, optical sensor unit is described. This optical sensor unit measures hemoglobin concentration and hemoglobin oxygen saturation which is required in determining the oxygen supply rate. Measurement of the hemoglobin concentration and the hemoglobin oxygen saturation requires measurement of absorbance at two or more wavelengths, and FIG. 13C shows an embodiment wherein the measurement is conducted at two wavelengths by two light sources 33 and 34 and a detector 35.

At the optical sensor unit are located ends of two optical fibers 31 and 32. The optical fiber 31 is the optical fiber for irradiation the light, and the optical fiber 32 is the optical fiber for receiving the light. As shown in FIG. 13C, the optical fiber 31 is connected to branch fibers 31 a and 31 b respectively having light emitting diodes 33 and 34 at their end. The light emitting diodes 33 and 34 emit light of different wavelengths. The light-receiving optical fiber 32 has a photodiode 35 at its end. The light emitting diode 33 produces a light beam at a wavelength of 810 nm, and the light emitting diode 34 produces a light beam at a wavelength of 950 nm. The wavelength of 810 nm is the equal absorption wavelength at which molar extinction coefficient of the oxygenated hemoglobin equals to that of the reduced (deoxygenated) hemoglobin, and the wavelength of 950 nm is the wavelength at which the oxygenated hemoglobin and the reduced hemoglobin exhibits a significantly different molar extinction coefficient.

The two light emitting diodes 33 and 34 are actuated in a time-sharing manner, and the light beam emitted from these diodes 33 and 34 are directed through the light irradiation optical fiber 31 to the finger of the subject. The light beam directed to the finger is reflected by the skin of the finger and enters the light-receiving optical fiber 32 to be detected by the photodiode 35. When the light beam directed to the finger is reflected by the finger skin, a part of the light enters through the skin into the interior of the tissue to be absorbed by the hemoglobin in the blood flowing through the capillaries. The data detected by the photodiode 35 is reflectivity R, and the absorbance is approximated by log (1/R). The light beams at the wavelength of 810 nm and 950 nm are respectively emitted, and the reflectivity R and the log(1/R) are determined for each wavelength to thereby obtain absorbance A1 at the wavelength of 810 nm and absorbance A2 at the wavelength of 950 nm.

The absorbance $A_1$ and the absorbance $A_2$ are represented by the following equations when concentration of the reduced hemoglobin is represented by [Hb] and concentration of the oxygenated hemoglobin is represented by [HbO$_2$].

$$A_1 = a \times ([Hb] \times A_{Hb}(810nm) + [HbO_2] \times A_{HbO_2}(810nm))$$
$$= a \times ([Hb] + [HbO_2]) \times A_{HbO_2}(810nm)$$

$$A_2 = a \times ([Hb] \times A_{Hb}(950nm) + [HbO_2] \times A_{HbO_2}(950nm))$$
$$= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm))$$

In the equation, $A_{Hb}$ (810 nm) and $A_{Hb}$ (950 nm) are molar extinction coefficients of the reduced hemoglobin, and $A_{HbO2}$ (810 nm) and $A_{HbO2}$ (950 nm) are those of the oxygenated hemoglobin, and these molar extinction coefficients are known for each wavelength. "a" is a proportionality factor. From the above-described equation, concentration of the ($[Hb] + [HbO_2]$) and saturation of the hemoglobin with the oxygen ($[HbO_2] / ([Hb] + [HbO_2])$) may be calculated as described below.

$$[Hb] + [HbO_2] = \frac{A_1}{a \times A_{HbO_2}(810nm)}$$

$$\frac{[HbO_2]}{[Hb] + [HbO_2]} = \frac{A_2 \times A_{HbO_2}(810nm) - A_1 \times A_{Hb}(950nm))}{A_1 \times (A_{HbO_2}(950nm) - A_{Hb}(950nm))}$$

In this example, an embodiment wherein the hemoglobin concentration and the hemoglobin oxygen saturation are determined by measuring the absorbance at two wavelengths were described. It is to be noted that the absorbance may also be measured at three or more wavelengths to thereby improve the measurement accuracy by reducing the influence of inhibitory factors.

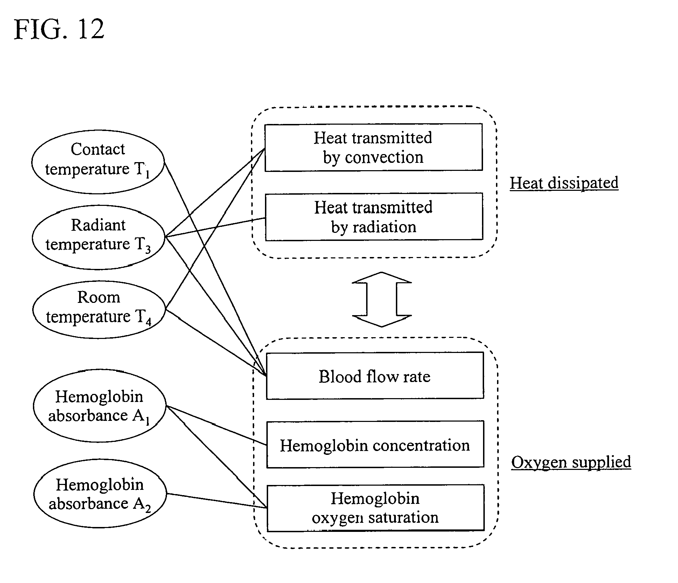

The parameter $x_i$ (i = 1, 2, 3, 4, 5) required for estimating the blood glucose level are calculated from the measurements obtained by the sensor having the constitution as described above. More specifically, the parameters $x_i$ are as described below (in the equation, $a_1$ to $a_5$ are proportionality factors). The relation between the parameter and the measurements are shown in FIG. 12.

Parameter proportional to the heat radiation:

$$x_1 = a_1 \times (T_3)^4$$

Parameter proportional to the heat convection:

$$x_2 = a_2 \times (T_4 - T_3)$$

Parameter proportional to the hemoglobin concentration:

$$x_3 = a_3 \times \left( \frac{A_1}{a \times A_{HbO_2}(810nm)} \right)$$

Parameter proportional to the hemoglobin saturation:

$$x_4 = a_4 \times \left( \frac{A_2 \times A_{HbO_2}(810nm) - A_1 \times A_{Hb}(950nm))}{A_1 \times (A_{HbO_2}(950nm) - A_{Hb}(950nm))} \right)$$

Parameter proportional to the blood flow rate (the factor $B_1$ in the fitting function represented by equation (1) divided by the room temperature and the finger surface temperature):

$$x_5 = a_5 \times (B_1 / (T_3 - T_4))$$

Next, normalized parameters are calculated from the average and the standard deviation of the parameters $x_i$

calculated from the data collected from a large number of normal donors and diabetes patients. More specifically, the normalized parameters $X_i$ (i = 1, 2, 3, 4, 5) are calculated from the parameters $X_i$ by the following equation.

$$X_i = \frac{x_i - \bar{x}_i}{SD(x_i)}$$

$x_i$ : parameters
$\bar{x}_i$ : average of the parameters
$SD(x_i)$ : s tan dard deviation of the parameters

The glucose concentration to be finally displayed is calculated by using the thus determined five normalized parameters. The program required for such arithmetic processing are stored in the ROM in the microprocessor installed in the device. The memory region required for the arithmetic processing is secured in the RAM also installed in the device. The result of the arithmetic processing is displayed on the liquid crystal display unit.
The ROM includes the function for determining the glucose concentration C as a program constituent required for the arithmetic processing. This function has been determined as described below. First, C is expressed by the following equation (3). In this equation, $a_i$ (i = 0, 1, 2, 3, 4, 5) are factors preliminarily determined from the measured data by the procedure as described below.

(1) A multiple regression equation showing the relation between the normalized parameters and the glucose concentration C is prepared.
(2) Normal equation (simultaneous equations) relating to the normalized parameters is determined from the equation obtained by least square method.
(3) The values of the factors $a_i$ (i = 0, 1, 2, 3, 4, 5) are determined from the normal equation, and the determined values are introduced in the multiple regression equation.

First, the following regression equation (3) showing the relation between the glucose concentration C and the normalized parameters $X_1$, $X_2$, $X_3$, $X_4$, $X_5$ is prepared.

$$C = f(X_1, X_2, X_3, X_4, X_5)$$
$$= a_0 + a_1 X_1 + a_2 X_2 + a_3 X_3 + a_4 X_4 + a_5 X_5) \qquad \cdots (3)$$

Next, least square method is used to determine the multiple regression equation wherein error from the glucose concentration $C_i$ measured by enzyme electrode method is minimized. When the residual sum of squares is D, D is represented by the following equation.

$$D = \sum_{i=1}^{n} d_i^2$$
$$= \sum_{i=1}^{n} (C_i - f(X_{i1}, X_{i2}, X_{i3}, X_{i4}, X_{i5}))^2$$
$$= \sum_{i=1}^{n} \{C_i - (a_0 + a_1 X_{i1} + a_2 X_{i2} + a_3 X_{i3} + a_4 X_{i4} + a_5 X_{i5})\}^2$$

The residual sum of squares D is minimum when this equation partially differentiated with $a_0$, $a_1$, ..., $a_5$ is zero. This gives the following equations.

$$\frac{\partial D}{\partial a_0} = -2\sum_{i=1}^{n}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_1} = -2\sum_{i=1}^{n} X_{i1}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_2} = -2\sum_{i=1}^{n} X_{i2}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_3} = -2\sum_{i=1}^{n} X_{i3}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_4} = -2\sum_{i=1}^{n} X_{i4}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_5} = -2\sum_{i=1}^{n} X_{i5}\{C_i - (a_0 + a_1 X_{i1} + a_2 X_{i2} + a_3 X_{i3} + a_4 X_{i4} + a_5 X_{i5})\} = 0$$

When the average of C and $X_1$ to $X_5$ are $C_{mean}$ and $X_{1mean}$ to $X_{5mean}$, $X_{imean} = 0$ (i = 1 to 5), and this gives the following equation.

$$a_0 = C_{mean} - a_1 X_{1mean} - a_2 X_{2mean} - a_3 X_{3mean} - a_4 X_{4mean} - a_5 X_{5mean}$$
$$= C_{mean} \qquad\qquad \cdots(4)$$

The variation and covariation between the normalized parameters are represented by $S_{ij}$ of the following equation, and covariation between the normalized parameters $X_i$ (i = 1 to 5) and C is represented by $S_{iC}$ of the following equation.

$$S_{ij} = \sum_{k=1}^{n}(X_{ki} - X_{imean})(X_{kj} - X_{jmean}) = \sum_{k=1}^{n} X_{ki} X_{kj} \quad (i, j = 1,2,..5)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,..5)$$

Organization of the equations as described above gives the following simultaneous equations (normal equation), and $a_1$ to $a_5$ are obtained by solving these equations.

$$a_1 S_{11} + a_2 S_{12} + a_3 S_{13} + a_4 S_{14} + a_5 S_{15} = S_{1C}$$

$$a_1 S_{21} + a_2 S_{22} + a_3 S_{23} + a_4 S_{24} + a_5 S_{25} = S_{2C}$$

$$a_1 S_{31} + a_2 S_{32} + a_3 S_{33} + a_4 S_{34} + a_5 S_{35} = S_{3C}$$

$$a_1 S_{41} + a_2 S_{42} + a_3 S_{43} + a_4 S_{44} + a_5 S_{45} = S_{4C}$$

$$a_1 S_{51} + a_2 S_{52} + a_3 S_{53} + a_4 S_{54} + a_5 S_{55} = S_{5C}$$

Constant term $a_0$ is determined by using equation (4). The thus determined $a_i$ (i = 0, 1, 2, 3, 4, 5) are accommodated in the ROM at the time of the device production. In the actual measurement using the device, the glucose concentration C is calculated by substituting the normalized parameters $X_1$ to $X_5$ in the regression equation (3).

Next, a typical embodiment of calculating the glucose concentration is described. The factors of the regression equation (3) have been determined by using a large number of data obtained by measuring normal donors and diabetes patients. The equation for calculating the glucose concentration as described below is accommodated in the ROM of the microprocessor.

$$C = 99.4 + 18.3 \times X_1 - 20.2 \times X_2 - 23.7 \times X_3 - 22.0 \times X_4 - 25.9 \times X_5$$

$X_1$ to $X_5$ are parameters $x_1$ to $x_5$ which have been normalized. When the parameters are postulated to be normally distributed, 95% of the normalized parameters are values within the range of -2 to +2.

When normalized parameters $X_1$ = -0.06, $X_2$ = +0.04, $X_3$ = +0.05, $X_4$ = -0.12, and $X_5$ = +0.15 are substituted into the equation as described above as an example of the normal donor measurement, C is 94.7 mg/dl. When normalized parameters $X_1$ =+1.15, $X_2$ = -1.02, $X_3$ = -0.83, $X_4$ = -0.91, and $X_5$ = -1.34 are substituted into the equation as described above as an example of the diabetes patient measurement, C is 210.4 mg/dl.

FIG. 10 shows comparison of the measurements obtained by the blood glucose measurement device of this Example and the measurements obtained by the conventional enzyme electrode method. The correlation was 0.92 indicating that the blood glucose measurement device of this Example is capable of measuring the blood glucose level at a high precision.

Example 3

This Example illustrates an embodiment in which the measurement device of the present invention is used in estimating metabolic rate.

Based on the thermal control mechanism of human body, the heat produced by metabolism (metabolic heat production) is equal to the sum of the heat accumulated in the body (accumulated heat) and the heat dissipated from the body (dissipated heat). This gives the following equation.

(Metabolic rate of the entire body [metabolic heat production]) = (heat accumulated in the entire body) + (heat dissipated from the entire body)    (5)

When body at site $r_i$ has an internal temperature T, the site $r_i$ has a tissue temperature $T_T$, and the site $r_i$ has a heat capacity $\alpha_i$, the heat accumulated in the entire body will be the total of the heat accumulated in each parts, and such heat accumulated in the entire body can be represented by the following equation.

$$\text{Accumulated heat of the entire body} = \sum_{i} \alpha_i \left\{ T(\mathbf{r}_i) - T_T(\mathbf{r}_i) \right\}$$

While the equation as described above deals with the heat of each part of the body, temperature of arterial blood Ta in the body core may be used as an effective value representing the temperature of the body core, and body temperature $T_c$ may be used as an effective value representing the temperature of the body tissue. Also with regard to the heat capacity, $\alpha$ may be used as an effective value representing the heat capacity of the entire body. This value $\alpha$ depends on body composition, density, body weight, and the like. Accordingly, the heat accumulated in the entire body can be represented by the following equation.

$$\text{Heat accumulated in the entire body} = \alpha(Ta - Tc) \quad \dots (6)$$

Heat dissipation is generally accomplished by conduction, radiation, convection, and evaporation. The conduction and the radiation, however, is small and negligible when a person is wearing cloths. The evaporation would also be negligible in an indoor climate in which the temperature is controlled to prevent perspiration. Accordingly, the convection between the body surface and the air is solely considered, and in this case, when the body surface at site $r_j$ has a skin temperature $T_s$, the body surface at site $r_j$ is in contact with an outer temperature $T_{OUT}$, and the body surface at site $r_j$ has a convectional thermal conductivity $\beta_j$, the heat dissipation from the entire body will he the sum of the heat dissipation from the body surface and this can be represented by the following equation.

$$\text{Accumulated heat of the entire body} = \sum_{j} \beta_j \left\{ T_S(\mathbf{r}_j) - T_{OUT}(\mathbf{r}_j) \right\}$$

While the equation as described above deals with the heat of each part of the body, finger skin temperature $T_{FS}$ may be used as an effective value representing the body surface temperature, and room temperature $T_R$ may be used as an effective value representing the outer temperature. Also with regard to the convectional thermal conductivity, $\beta$ may be used as an effective value representing the convectional thermal conductivity of the entire body surface. This value $\beta$ depends on surface area, clothing, and the like of each person. Accordingly, the heat dissipation from the entire body can be represented by the following equation.

$$\text{Heat dissipation from the entire body} = \beta(T_{FS} - T_R) \quad \dots (7)$$

In the present invention, the finger is used for the site representing the heat dissipation of the entire body since the finger is a site sensitive to the change of outer temperature, and it undergoes a considerable change of the skin temperature and the heat dissipation. This site also has less muscles, and since local heat production is at a low level, this site vividly reflects the situation of the entire body.

From equations (5), (6), and (7), the metabolic rate of the entire body can be represented by the following equation.

$$\text{(Metabolic rate of the entire body [heat production ])} = \alpha(T_a - T_c) + \beta(T_{FS} - T_R) \quad \dots (8)$$

Based on the law of heat conservation of the finger, the heat coming into the finger should be balanced with the heat dissipating from the finger. With regard to the finger tissue, when the artery in the core of the finger has a temperature $TF_a$, the finger tissue has a convectional thermal conductivity $\lambda$, the finger tissue has a blood flow rate $\omega_b$, the blood has a specific heat $C_b$, and the finger surface has a convectional thermal conductivity $\kappa$, the law of heat conservation for the finger can be represented by the following equation. In this equation, $\chi$ is a factor of proportionality.

$$\lambda(\chi\omega_b cbTF_a - T_{FS}) = \kappa(T_{FS} - T_R) \qquad \cdots (9)$$

The left hand side of the equation represents the heat transfer from the artery to the tissue near the finger surface, and this is in accordance with the biological heat transfer equation of Pennes (Pennes H.H., "Analysis of tissue and arterial blood temperatures in the resting human forearm" J. applied physiology, 1, 93-122(1984).

When the arterial temperature $T_{Fa}$ in the finger core is proportional to the arterial temperature $T_a$ in the body core, and $T_{Fa}$ is postulated to be equal to $\sigma T_a + \tau$, equation (9) can be expressed as follows.

$$T_a = \frac{1}{\sigma\chi c_b}\left\{\frac{\kappa}{\lambda}(T_{FS} - T_R)/\omega_b + T_{FS}/\omega_b\right\} - \frac{\tau}{\sigma} \qquad \cdots(10)$$

Substitution of this equation (10) in equation (8) gives equation (11).

$$Metabolic\ rate\ of\ the\ entire\ body\ [heat\ production]$$

$$= \frac{\alpha}{\sigma\chi c_b}\left\{\frac{\kappa}{\lambda}(T_{FS} - T_R)/\omega_b + T_{FS}/\omega_b\right\} + \beta(T_{FS} - T_R) - \alpha T_c - \frac{\alpha\tau}{\sigma}$$

$$= a(T_{FS} - T_R)/\omega_b + bT_{FS}/\omega_b + c(T_{FS} - T_R) + dT_c + e \quad \cdots(11)$$

$$a = \frac{\alpha}{\sigma\chi c_b}\frac{\kappa}{\lambda}, \quad b = \frac{\alpha}{\sigma\chi c_b}, \quad c = \beta, \quad d = -\alpha, \quad e = -\frac{\alpha\tau}{\sigma}$$

Use of equation (11), however, requires preliminary determination of the proportionality constants a to e, which are different from person to person. For such determination, metabolic rate of each person may be measured, and the proportionality factors may be determined from the measured metabolic rate and equation (11) by multiple regression analysis.

The metabolic rate may be determined by the model equation as described above, and such determination may be conducted by measuring the finger surface temperature $T_{FS}$ as an effective value of the body surface temperature, the room temperature $T_R$, the body temperature $T_c$ as an effective value of the body tissue temperature, and the blood flow rate $\omega_b$, and using such measurements with the separately determined proportionality factors. While fingertip has been used as a representative measurement site, other sites on the body surface may be used instead of the fingertip, and the temperature corresponding to the tissue temperature in the body core can be obtained by measuring a temperature measurement site which is different from those that had been used in obtaining an effective value of the body surface temperature such as under the tongue, armpit, and rectum. The temperature measurement site used to obtain the effective value representing the body surface temperature is not limited to the finger surface, and other sites on the limb may also be used.

Since measurement of the metabolic rate requires a large scale apparatus, the metabolic rate corresponding to the glucose concentration may be determined by using the relation between the concentration of the blood glucose which is the human energy source and the metabolic rate. The relation between the metabolic rate and the glucose concentration in the entire body is, when the heat produced by the glucose heat production in each body site $r_k$ is QG $(r_j)$, and the heat produced by the heat production by substances other than the glucose such as fat and amino acid is $Q\Pi(r_j)$, such that the metabolic rate of the entire body is the total sum of the heat generated in each site of the body, and this and can be represented by the following equation.

$$(Metabolic\ rate\ of\ the\ entire\ body\ [heat\ produced] = \sum_k\{QG(\mathbf{r}_k) + Q\Pi(\mathbf{r}_k)\} \qquad \cdots(12)$$

While equation (12) deals with the heat of each body site, when it is postulated that the average intracellular glucose concentration in the entire body Gc is proportional to the metabolic rate by the intracellular glucose, and the average intracellular concentration of the substances other than glucose in the entire body $\Pi$ is proportional to the metabolic rate by such non-glucose substances, and the proportionality factors are A and B, respectively, the metabolic rate of the entire body can be expressed by the following equation.

$$\text{Metabolic rate of the entire body} = AGc + B\Pi \quad \ldots(13)$$

Equation (11) and equation (13) give the following equation.

$$AG_c = a\left(T_{FS} - T_R\right)/\omega_b + bT_{FS}/\omega_b + c\left(T_{FS} - T_R\right) + dT_c + e - B\Pi \quad \cdots(14)$$

The metabolic rate $AG_c$ corresponding to the blood glucose may be determined by the model equation as described above, and such determination may be conducted by measuring the finger surface temperature $T_{FS}$ as an effective value representing the body surface temperature, the room temperature $T_R$, the body temperature $T_c$ as an effective value of the body tissue temperature, and the blood flow rate $\omega_b$, and using such measurements with separately measured proportionality factors.

When a measurement with higher accuracy is required, tissue oxygen saturation may be taken into consideration. Since the heat dissipation from the entire body is proportional to the amount of oxygen used, the relation: Gc + $\Pi \propto$ [O$_2$ consumption] is found. Since the tissue oxygen saturation StO$_2$ is equivalent to the O$_2$ consumption, this relation can be expressed by the following equation.

$$StO_2 = a'Gc + b'\Pi \quad \ldots(15)$$

Equation (14) and equation (15) give the following equation (16).

$$\left(\frac{Ab' - a'B}{b'}\right)G_c = a\left(T_{FS} - T_R\right)/\omega_b + bT_{FS}/\omega_b + c\left(T_{FS} - T_R\right) + dT_c + e - \frac{B}{b'}StO_2 \quad \cdots(16)$$

The metabolic rate ((ab' - a'b)/b') Gc corresponding to the blood glucose may be determined by the model equation as described above, and such determination may be conducted by measuring the finger surface temperature $T_{FS}$ as an effective value representing the body surface temperature, the room temperature $T_R$, the body temperature Teas an effective value of the body tissue temperature, the blood flow rate $\omega_b$, and the tissue oxygen saturation StO$_2$, and using such measurements with separately measured proportionality factors.

Organization of the coefficients of equation (16) gives the following equation (17), and calculation of the glucose concentration is enabled.

$$G_c = a\left(T_{FS} - T_R\right)/\omega_b + bT_{FS}/\omega_b + c\left(T_{FS} - T_R\right) + dT_c + eStO_2 + f \quad \cdots(17)$$

$$a = \left(\frac{b'}{ab' - a'b}\right)\frac{\alpha}{\sigma\chi c_b}\frac{\kappa}{\lambda}, \quad b = \left(\frac{b'}{ab' - a'b}\right)\frac{\alpha}{\sigma\chi c_b}$$

$$c = \left(\frac{b'}{ab' - a'b}\right)\beta, \quad d = -\left(\frac{b'}{ab' - a'b}\right)\alpha$$

$$e = -\left(\frac{b'}{ab' - a'b}\right)\frac{b}{b'}, \quad f = -\left(\frac{b'}{ab' - a'b}\right)\frac{\alpha\tau}{\sigma}$$

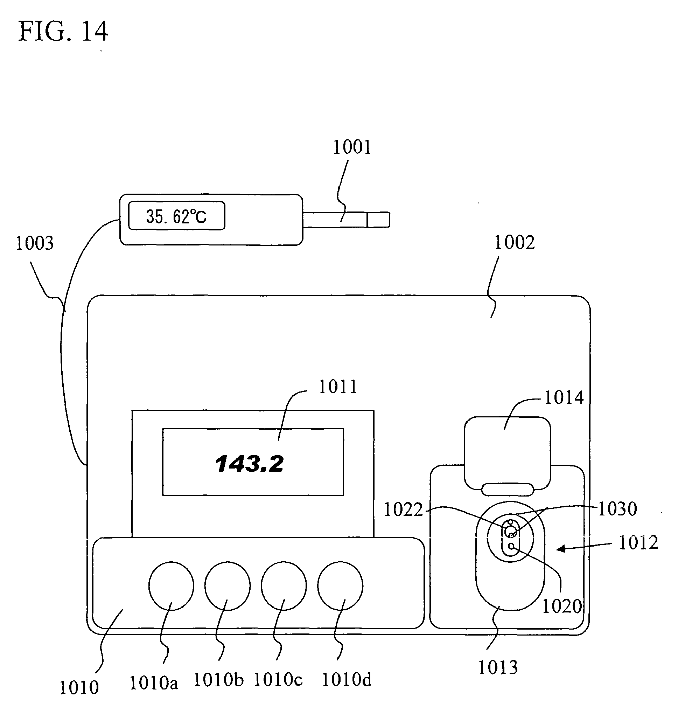

FIG. 14 is a top view of a metabolism meter according to the present invention. In this device, skin of the finger pad is used for the body surface. Use of other body surface is also possible in view of the measurement principle.

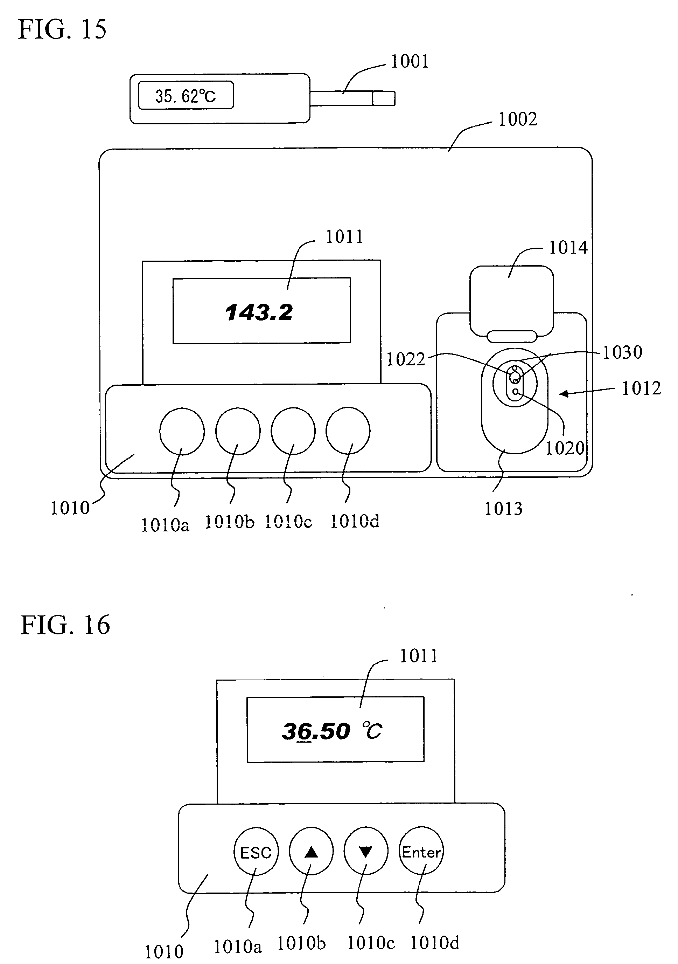

The device comprises a body temperature measurement unit 1001 which measures the body temperature, and main unit 1002 which measures room temperature, finger surface temperature, blood flow rate, and tissue oxygen saturation, and the body temperature measurement unit 1001 and the main unit 1002 are connected by a wiring 1003. In this embodiment, the body temperature measurement unit 1001 is connected to the main unit 1002, and the body temperature data measured in the body temperature measurement unit 1001 is transmitted to the main unit 1002. However, a commercially available clinical thermometer may be used for the measurement of the body temperature, and the data obtained by such thermometer may be used for the input of the data at an operation unit 1010 of the main unit 1002. The measurement of the blood flow rate and the tissue oxygen saturation may also be carried out by a commercially available device, and the resulting data of the blood flow rate and the tissue oxygen saturation may be used for the input of the data at an operation unit 1010 of the main unit 1002. FIG. 15 shows top view of the metabolic rate measuring device of the present invention when the body temperature and the blood flow rate are measured by commonly available devices.

Input of the body temperature and other data may be conducted on the screen, for example, the one shown in FIG. 16. In this embodiment, 36.50°C is displayed as the preset value, and the one's place digit is underscored to indicate that this place is to be entered. The number is changed by using operation buttons 1010b and 1010c. More specifically, the number displayed can be increased by pressing the operation button 1010b, and the number can be reduced by pressing the operation button 1010c so that the number displayed will be consistent with the body temperature measured. The digit in the one's place of the body temperature is then confirmed by pressing operation button 1010d. This step is repeated for the first and the second digits to the right of the decimal point to complete the data input of the body temperature. When the operation button 1010d is pushed by error, operation button 1010a may be pressed to redo the body temperature input.

The top surface of the device has the operation unit 1010, a measurement unit 1012 where the finger to be measured is to be placed, and display unit 1011 for displaying the measurement results and conditions and measurements of the device. The operation unit 1010 is provided with the four push buttons 1010a to 1010d. The measurement unit 1012 is provided with a cover 1014, and when this cover 1014 is opened (FIG. 15 shows the state with the cover opened), a finger rest 1013 with oblong contour will be found. In the finger rest 1013 is provided an open end 1022 of a radiation temperature sensor unit, an optical sensor unit 1030, and a temperature sensor unit 1020. The optical sensor unit 1030 and the temperature sensor unit 1020 has a structure which is basically the same that of FIG. 13.

Arithmetic unit of the device calculates five physiological parameters (body temperature, room temperature, finger surface temperature, blood flow rate, and tissue oxygen saturation), and conversion to the metabolic rate to be finally displayed is conducted by using these five physiological parameters.

Next, an embodiment of calculating the metabolic rate is described. Coefficients a to e of the equation (11) are preliminarily determined from the data collected for a large number of people, and the following equation used in calculating the metabolic rate has been accommodated in the ROM of the microprocessor.

$$Metabolic\ rate = 439 - 128T_c + 893(T_{FS} - T_R) - 773(T_{FS} - T_R)/\omega_b - 173T_{FS}/\omega_b$$

The measurements required in this stage are the body temperature $T_c$, the finger surface temperature $T_{FS}$, the room temperature $T_R$, and the blood flow rate $\omega_b$, and therefore, these measurements required for the measurement of the metabolic rate can be obtained by the device shown in FIG. 14 or 15 having the sensor unit of FIG. 13 incorporated therein.

As an example, the measured data including $T_c$ = 36.46, $T_{FS} - T_R$ = 7.52, $(T_{FS}-T_R)/\omega_b$ = 11.30, and $T_{FS}/\omega_b$ = 47.79 were substituted into the equation as described above, and this gave a metabolic rate of 8.5 kJ. In this case, the metabolic rate obtained by the indirect calorimetry of the closed circuit system was 8.6 kJ. When the measured data when the metabolic rate obtained by the indirect calorimetry of the closed circuit system was 8.0 kJ ($T_c$ = 36.56, $T_{FS} - T_R$ = 7.96, $(T_{FS} - T_R)/\omega_b$ = 12.16, and $T_{FS}/\omega_b$ = 48.99) were substituted into the equation as described above, a metabolic rate of 8.1 kJ was obtained.

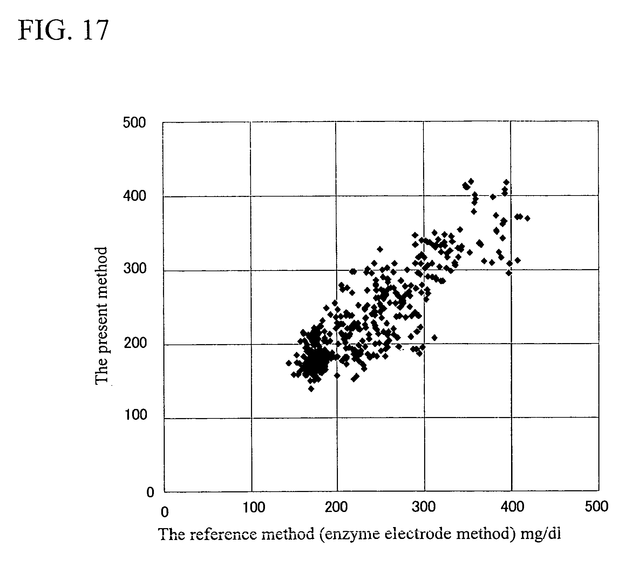

FIG. 17 is a view wherein the measured values of a plurality of subjects are plotted in a graph wherein vertical axis represents the metabolic rate calculated by the method of the present invention and the horizontal axis expresses the metabolic rate obtained by the indirect calorimetry of the closed circuit system. A good correlation is obtained when the body temperature, the finger temperature, and the blood flow rate were measured and the metabolic rate was calculated by the method of the present invention (correlation coefficient = 0.82).

Next, a typical embodiment of calculating the glucose concentration is described. The factors of the equation (17) have been determined by using a large number of data obtained by measuring a large number of people. The equation for calculating the glucose concentration as described below is accommodated in the ROM of the microprocessor.

$$Gc = -360.9 + 7.9T_c + 14.8(T_{FS} - T_R) - 5.2(T_{FS} - T_R)/\omega_b + 1.3T_{FS}/\omega_b + 152.7StO_2$$

As an example, the measured data including $T_c$ = 36.71, $T_{FS}$-$T_R$ = 5.18, $(T_{FS}$-$T_R)/m_b$ = 8.39, $T_{FS}/w_b$ = 50.10, and $StO_2$ = 0.46 were substituted into the equation as described above, and this gave a value of 97.5. In this case, the blood glucose concentration was 108.6 g/dl. When the measured data when the blood glucose concentration was 120.1 mg/dl ($T_c$ = 36.47, $T_{FS}$-$T_R$ = 7.91, $(T_{FS}$-$T_R)/\omega_b$ = 15.64, $T_{FS}/\omega_b$ = 62.92, and $StO_2$ = 0.45) were substituted into the equation as described above, this gave a value of 113.5 mg/dl.

When the calculated glucose concentration was multiplied by the coefficient A of equation (13), a metabolic rate corresponding to the blood glucose concentration could be calculated.

In this example, the metabolic rate determined was the one corresponding to the blood glucose concentration. However, the metabolic rate is not limited to the one determined by glucose, and the metabolic rate may also be determined by using neutral fat and/or cholesterol. In such a case, a calculation equation may be prepared from the blood concentration of such neutral fat and/or cholesterol and the measurement of the metabolic rate, and the metabolic rate corresponding to the neutral fat or cholesterol concentration can be determined from the thus prepared equation.

Since the trend of the accumulated measurements of the calculated metabolic rate or the metabolic rate corresponding to the glucose concentration or the blood glucose concentration is different between the normal donors and the patients suffering from metabolic diseases such as arteriosclerosis, heart disease, and impaired glucose tolerance, the calculated value can be used as an index for grasping the disease condition. Apprehension of the trend of the metabolic rate may be facilitated by displaying the accumulated metabolic rate data in the time sequence.

**Claims**

1.  A blood glucose measurement device for measuring the blood glucose level, comprising:

    a heat measurement unit for measuring a plurality of temperatures from a body surface to obtain information used in calculating the amount of heat transmitted by convection and radiation in heat dissipation from the body surface;
    an oxygen content measuring unit for obtaining information on the blood oxygen content;
    a storage unit for storing the relations between parameters respectively corresponding to said plurality of temperatures and said blood oxygen content and the blood glucose level;
    an arithmetic unit for converting the plurality of measurements received from said heat measurement unit and said oxygen content measuring unit respectively into said parameters, and calculating the blood glucose levels by applying said parameters to said relations stored in said storage unit; and
    a display unit for displaying the result obtained in the arithmetic unit,

    wherein
    said oxygen content measuring unit comprises a unit for measuring the blood flow rate to obtain information on the blood flow rate; and an optical measurement unit for obtaining the blood hemoglobin concentration and the hemoglobin oxygen saturation, and
    said blood flow rate measuring unit comprises a member to be brought in contact with the body surface; a temperature sensor provided in contact with said body surface contacting member at a surface opposite to the side to be brought in contact with the body surface; a metal block; and a thermal connection member for thermally connecting said temperature sensor and said metal block.

2.  A blood glucose measurement device for measuring the blood glucose level, comprising:

an ambient temperature measurement device for measuring the ambient temperature;

a body surface contacting unit to be brought in contact with a body surface;

a radiant heat sensor for measuring heat radiated from the body surface;

a temperature sensor provided in contact with said body surface contacting member on a surface opposite to the side to be brought in contact with the body surface;

a metal block;

a thermal connection member for thermally connecting said temperature sensor and said metal block;

a light source for emitting at least two beams respectively having different wavelengths to said body surface contacting member;

a photodetector for sensing the beam that had been directed to said body surface;

an arithmetic unit having a conversion section for converting outputs from said ambient temperature measurement device, said radiant heat detector, said temperature sensor, and said photodetector respectively to the corresponding parameters, and an arithmetic section which preliminarily stores the relation between said parameters and the blood glucose level and which calculates the blood glucose level by applying said parameters to said relationships; and

a display unit for displaying the output of the arithmetic section.

3. A metabolic rate measurement device comprising:

an ambient temperature measurement device for measuring the ambient temperature;

a first temperature measurement device for measuring the temperature of a first body surface site of the subject;

a second temperature acquiring unit for acquiring the temperature of a second body surface site of the subject which is different from the first body surface site;

a blood flow rate acquiring unit for acquiring information on the blood flow rate of said first site;

a storage unit for storing the relation between said ambient temperature, said temperature of the first site, said temperature of the second site, and said blood flow rate and the metabolic rate;

an arithmetic unit for calculating the metabolic rate by applying the ambient temperature measured by said ambient temperature measurement device, the temperature of the first site measured by said first temperature measurement device, the temperature of the second site measured by said second temperature measurement device, and the blood flow rate acquired by said blood flow rate acquiring unit to the relation stored in said storage unit; and

a display unit for displaying the results calculated in the arithmetic section,

wherein said blood flow rate acquiring unit comprises a body surface contacting section which contacts with said first body surface site, a temperature sensor provided in contact with said body surface contacting member on a surface opposite to the side to be brought in contact with the body surface, a metal block, and a thermal connection member for thermally connecting said temperature sensor and said metal block.

4. The device of claim 3, wherein said second temperature acquiring unit comprises:

a thermometer, wherein an output of the thermometer is supplied to said arithmetic unit; and/or

an operation unit for numerically entering the body temperature.

5. The device of claim 3, wherein
the device further comprises an oxygen saturation acquiring unit, and said storage unit stores the relation between said ambient temperature, said temperature of the first site, said temperature of the second site, said blood flow rate, and said oxygen saturation and the metabolic rate, and
said arithmetic unit calculates the metabolic rate by applying the ambient temperature measured by said ambient temperature measurement device, the temperature of the first site measured by said first temperature measurement device, the temperature of the second site acquired by said second temperature acquiring unit, the blood flow rate acquired by said blood flow rate acquiring unit, and the oxygen saturation acquired by said oxygen saturation acquiring unit to the relations stored in said storage unit.

6. A metabolic rate measurement device comprising:

an ambient temperature measurement device for measuring the ambient temperature;

a body surface contacting unit which contacts with a first body surface site;

a cylindrical member which is in contact with said body surface contacting unit and which is open at one end;

a radiant heat thermometer for measuring the radiant heat emitted from said first site, the radiant heat thermometer being provided near the end of said cylindrical member other than said open end;

a blood flow rate acquiring unit for acquiring information on the blood flow rate of said first site, said unit comprising a temperature sensor provided in contact with said body surface contacting unit at a surface opposite to the side to be brought in contact with the body surface, a metal block, and a thermal connection member for thermally connecting said temperature sensor and said metal block, and said information on the blood flow rate of said first site is acquired from a temperature change of the body surface contacting unit after being brought in contact with the body surface first site;

a light source which emits at least two light beams each having different wavelengths to said end of said cylindrical member;

a photodetector for detecting the light beam which has interacted with said body surface;

a second site temperature acquiring unit for acquiring temperature of the second site which is different from said first site

an arithmetic unit wherein the ambient temperature measured by said ambient temperature measurement device, the temperature of the first site measured by said first temperature measurement device, the temperature of the second site acquired by said second temperature acquiring unit, the blood flow rate acquired by said blood flow rate acquiring unit, and the results measured by said photodetector are applied to the preliminarily stored relations between these values and the metabolic rate to thereby calculate the metabolic rate; and

a display unit for displaying the output of the arithmetic section.

7. The device of any preceding claim, wherein said thermal connection member has a thermal conductivity lower than that of said metal block.

8. The device of any preceding claim, wherein said metal block has a heat capacity of at least $3.0 \times 10^{-3}$ (KJ/K).

9. The device of claim 6, wherein said second temperature acquiring unit comprises:

a thermometer; and/or
an operation unit for numerically entering the body temperature.

10. The device of claim 3 or 6, wherein said first site is a site in the subject's limb, and said first temperature measurement device measures the temperature of the site in the limb.

# FIG. 1

Finger surface temperature

High blood flow rate

Low blood flow rate

Immersion    Taking out                    Time

## FIG. 2

201

Ta                Ta                Ta                          Ta

Thermal
conductivity        202
∝ ω

Tt

203

205

204

206   Tr

## FIG. 3

Ta   301

Thermal
conductivity        302
∝ ω
303

Tt

304

Tr

305

FIG. 4

Finger

$Ta$(Blood temperature)

C1  R1

Thermal conductivity
$\propto \omega \propto 1/R1$

R2

Tr

C2

R3

Sensor

**FIG. 5 A**

**FIG. 5 B**

FIG. 6

FIG. 7

FIG. 8

Finger

803

Mechanism for displaying optimal value information

502

503

506

504

501

505

510

Measurement of displacement

801

507

701

spring

spring

702

Measurement of pressure

802

## FIG. 9

FIG. 10

FIG. 11

FIG. 12

Contact temperature $T_1$

Radiant temperature $T_3$

Room temperature $T_4$

Hemoglobin absorbance $A_1$

Hemoglobin absorbance $A_2$

Heat transmitted by convection

Heat transmitted by radiation

Heat dissipated

Blood flow rate

Hemoglobin concentration

Hemoglobin oxygen saturation

Oxygen supplied

FIG. 13 A

FIG. 13 B

FIG. 13 C

FIG. 14

FIG. 15

FIG. 16

FIG. 17

European Patent Office

**EUROPEAN SEARCH REPORT**

Application Number

EP 05 02 8512

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 563 787 A (HITACHI LTD [JP]) 17 August 2005 (2005-08-17) * paragraph [0009] - paragraph [0044] * ----- | 1-10 | INV. A61B5/00 |
| A | EP 1 568 309 A (HITACHI LTD [JP]) 31 August 2005 (2005-08-31) * paragraph [0005] - paragraph [0012] * ----- | 1-10 | |
| A | EP 1 537 822 A (HITACHI LTD [JP]) 8 June 2005 (2005-06-08) * paragraph [0011] - paragraph [0044] * * figure 7 * ----- | 1-3,6 | |
| A | DE 44 23 663 A1 (MED SCIENCE GMBH [DE]) 11 January 1996 (1996-01-11) * column 4, line 60 - column 9, line 43 * ----- | 1-3,6 | |

TECHNICAL FIELDS
SEARCHED      (IPC)

A61B

The present search report has been drawn up for all claims

| Place of search | Date of completion of the search | Examiner |
|---|---|---|
| Munich | 14 November 2006 | Rivera Pons, Carlos |

EPO FORM 1503 03.82 (P04C01)

## ANNEX TO THE EUROPEAN SEARCH REPORT
## ON EUROPEAN PATENT APPLICATION NO.

EP 05 02 8512

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2006

| Patent document cited in search report | | Publication date | Patent family member(s) | | Publication date |
|---|---|---|---|---|---|
| EP 1563787 | A | 17-08-2005 | CN | 1657006 A | 24-08-2005 |
| | | | JP | 3557425 B1 | 25-08-2004 |
| | | | JP | 2005230124 A | 02-09-2005 |
| | | | US | 2005182311 A1 | 18-08-2005 |
| EP 1568309 | A | 31-08-2005 | CN | 1660014 A | 31-08-2005 |
| | | | JP | 3590053 B1 | 17-11-2004 |
| | | | JP | 2005237484 A | 08-09-2005 |
| | | | US | 2005187442 A1 | 25-08-2005 |
| EP 1537822 | A | 08-06-2005 | CN | 1623508 A | 08-06-2005 |
| | | | JP | 3590049 B1 | 17-11-2004 |
| | | | JP | 2005160782 A | 23-06-2005 |
| | | | US | 2005124868 A1 | 09-06-2005 |
| DE 4423663 | A1 | 11-01-1996 | CA | 2194348 A1 | 18-01-1996 |
| | | | CN | 1159151 A | 10-09-1997 |
| | | | WO | 9601075 A1 | 18-01-1996 |
| | | | EP | 0771168 A1 | 07-05-1997 |
| | | | JP | 10503944 T | 14-04-1998 |
| | | | KR | 271095 B1 | 01-12-2000 |
| | | | US | 5924996 A | 20-07-1999 |

EPO FORM P0459

For more details about this annex : see Official Journal of the European Patent Office, No. 12/82

**REFERENCES CITED IN THE DESCRIPTION**

**Patent documents cited in the description**

- JP 2005300962 A
- JP 3478346 B
- JP 7248267 A
- JP 2004329542 A

**Non-patent literature cited in the description**

- **PENNES, H. H.** Analysis of tissue and arterial blood temperatures in the resting human forearm. *J. Applied Physiology,* 1948, vol. 1 (2), 93-122
- **PENNES H.H.** Analysis of tissue and arterial blood temperatures in the resting human forearm. *J. applied physiology,* 1984, vol. 1, 93-122